# EUROPÄISCHE PATENTSCHRIFT

(11) **EP 3 161 426 B1**
(45) Veröffentlichungstag und Bekanntmachung des Hinweises auf die Patenterteilung: **18.03.2020**
(21) Anmeldenummer: 15732566.3
(22) Anmeldetag: 16.06.2015
(51) Int. Cl.: G01F 15/14, G01F 1/58, A61M 1/14, A61M 1/16

(54) **KASSETTENMODUL FÜR EINEN FLUSSMESSER**
CARTRIDGE MODULE FOR A FLOW METER
MODULE DE CARTOUCHE POUR UN DÉBITMÈTRE

(30) Priorität: 25.06.2014 DE 102014009444
(43) Veröffentlichungstag der Anmeldung: 03.05.2017
(73) Patentinhaber: Fresenius Medical Care Deutschland GmbH, 61352 Bad Homburg (DE)
(72) Erfinder: HEIDE, Alexander, 65817 Eppstein (DE); NIKOLIC, Dejan, 65824 Schwalbach am Taunus (DE)
(74) Vertreter: Dreyhsig, Jörg
(86) Internationale Anmeldenummer: PCT/EP2015/063427
(87) Internationale Veröffentlichungsnummer: WO 2015/197424

(56) Entgegenhaltungen:
- EP-A1- 2 169 359
- WO-A1-2008/056976
- WO-A1-2013/175547
- DE-A1-102010 003 642
- GB-A- 2 056 691
- US-A- 4 585 552
- US-A1- 2012 095 537

## Beschreibung

### Technisches Gebiet

Die Erfindung bezieht sich auf ein Kassettenmodul ausgeführt für einen Differenzflussmesser zum Messen einer Differenz zwischen einem ersten Flüssigkeitsstrom und einem zweiten Flüssigkeitsstrom. Das Kassettenmodul weist Kanäle für den ersten und den zweiten Flüssigkeitsstrom auf.

### Hintergrund

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Bei der Hämodialyse (HD) überwiegt der diffusive Stofftransport, bei der Hämofiltration (HF) liegt ein konvektiver Stofftransport über die Membran vor. Eine Kombination aus beiden Verfahren ist die Hämodiafiltration (HDF). Bei der Peritonealdialyse (PD) wird kein extrakorporaler Kreislauf benötigt und das Peritoneum als Kontaktmembran ausgenutzt.

Wegen der großen Austauschmengen besteht bei den genannten Verfahren, sowie auch bei der kontinuierlichen arterio-venösen HF, der kontinuierlichen veno-venösen HF und der Plasmafiltration (PF) die Notwendigkeit der exakten Bilanzierung von entzogener Flüssigkeit einerseits zur zugeführten Flüssigkeit andererseits und der zu ultrafiltrierenden Menge über die gesamte Behandlungszeit. Zum Stand der Technik gehören gravimetrische und volumetrische Bilanziersysteme.

Darüber hinaus sind auch Verfahren bekannt, die die Flüssigkeitsströme der in den Dialysator einfließenden Flüssigkeiten und der aus dem Dialysator ausfließenden Flüssigkeiten kontinuierlich messen und gegeneinander bilanzieren. Hierzu werden Durchflussmesssensoren oder Flussmesser verschiedener Ausgestaltungen verwendet.

Magnetische Flussmesser, die auch als elektromagnetische Flussmesser oder induktive Flussmesser bezeichnet werden, beruhen auf der Messung der Strömungsgeschwindigkeit einer leitfähigen Flüssigkeit durch ein bekanntes oder kontrolliertes magnetisches Feld durch Messung der induzierten elektrischen Spannung. Bei bekanntem Strömungsquerschnitt kann von der Strömungs-geschwindigkeit auf die Flussrate oder den Volumenstrom geschlossen werden, was von dem Begriff des Flüssigkeitsstromes im Folgenden umfasst sein soll. Eine elektrische Spannung entsteht in einem durchströmten Magnetfeld durch eine Ladungstrennung der in einer leitfähigen Flüssigkeit vorliegenden Ionen, die als induzierte Spannung gemessen werden kann. Die Spannungsmessung erfolgt typischerweise, indem die induzierte Spannung an einem Paar von Elektroden, die in elektrischem Kontakt zu der leitfähigen Flüssigkeit stehen, oder die kapazitiv mit der Flüssigkeit gekoppelt sind, abgeleitet wird. Diese Spannung ist proportional zu der Strömungsgeschwindigkeit und abhängig von der Magnetfeldstärke. Die Ladungstrennung erfolgt in senkrechter Richtung zu der Flussrichtung sowie zu der Richtung des Magnetfeldes. Das Magnetfeld eines magnetischen Flussmessers ist daher vorzugsweise senkrecht zur Flussrichtung in dem entsprechenden Flüssigkeitskanal angeordnet und das Elektrodenpaar zum Ableiten der induzierten elektrischen Spannung ist vorzugsweise senkrecht sowohl zu dem Magnetfeld als auch zur Flussrichtung in dem Flüssigkeitskanal angeordnet.

Ein typischer elektromagnetischer Flussmesser ist aus einer nichtmagnetischen und nicht magnetisierbaren Röhre aufgebaut, die auf der Innenseite mit elektrisch isolierendem Material ausgekleidet ist.

Typischerweise wird das magnetische Feld durch eine oder mehrere außerhalb der flüssigkeitsdurchströmten Röhre angeordnete Spulen erzeugt. Die durch den Flüssigkeitsstrom induzierte elektrische Spannung wird typischerweise durch einen Spannungsmesser bestimmt und das Ergebnis der Spannungsmessung wird einer Auswerteeinheit zugeführt zur Bestimmung des Flüssigkeitsstromes, d.h. der Flussrate oder des Volumenstroms, basierend auf der gemessenen Spannung.

Wird ein elektromagnetischer Flussmesser als Differenzflussmesser zum Messen einer Flussdifferenz zwischen einem ersten und einem zweiten fluidführenden Kanal ausgebildet, so durchdringt vorteilhaft ein gemeinsames Magnetfeld den ersten und den zweiten fluidführenden Kanal.

Entsprechen der erste und der zweite fluidführende Kanal einander hinsichtlich ihrer geometrischen Abmessungen, so gibt die Spannungsdifferenz zwischen einem an dem ersten fluidführenden Kanal angeordneten ersten Elektrodenpaar und einem an dem zweiten fluidführenden Kanal angeordneten zweiten Elektrodenpaar unmittelbar die Differenz zwischen dem Fluss in dem ersten Kanal und dem Fluss in dem zweiten Kanal an. Werden das erste und das zweite Elektrodenpaar in Reihe geschaltet, so kann diese Spannungsdifferenz unmittelbar abgegriffen werden.

Ein elektromagnetischer Differenzflussmesser ist vorteilhaft aus dem einen Kassettenmodul, in dem die fluidführenden Kanäle ausgebildet sind, die jeweils ein Elektrodenpaar aufweisen, einem Elektromagneten oder Permanentmagneten zum erzeugen eines Magnetfeldes zwischen den Elektrodenpaaren und einer Auswerteeinheit und zum Auswerten der Spannungen oder der Differenzspannung zwischen den Elektrodenpaaren aufgebaut.

Das Kassettenmodul, in dem die fluidführenden Kanäle ausgebildet sind, ist vorteilhaft als Einmalteil ausgebildet, beispielweise als Teil eines Dialysierflüssigkeitskreislaufes.

Als Teil eines Dialysierflüssigkeitskreislaufs kann das Kassettenmodul weitere Elemente des Dialysierflüssigleitskreislaufs aufweisen oder nicht.

Aus der DE 10 2010 003642 ist eine Kassette in einem Dialysesystem bekannt, wobei die Kassette einen Sensor zur Bestimmung einer Differenz eines ersten und eines zweiten Flüssigkeitsstroms aufweist. Dieser Sensor weist einen ersten Kanal für den ersten Flüssigkeitsstrom und einen zweiten Kanal für den zweiten Flüssigkeitsstrom auf. Die Erfinder der vorliegenden Erfindung haben erkannt, dass eine Temperaturdifferenz zwischen dem ersten fluidführenden Kanal und dem zweiten fluidführenden Kanal zu einer verminderten Genauigkeit des Differenzflussmessers führen kann.

Aus der WO 2013/175547 A1 ist ein Kassettenmodul für einen Differenzflussmesser bekannt, welches einen ersten und einen zweiten im Betrieb des Differenzflussmessers fluidführenden Kanal ausbildet. Dabei wird eine geometrische Verformung der Kanäle durch eine Temperaturdifferenz zwischen den Kanälen minimiert bzw. verhindert, indem zwischen dem ersten und dem zweiten Kanal eine Isolationsschicht zur thermischen Isolation der Kanäle vorgesehen ist.

Es ist daher die Aufgabe der vorliegenden Erfindung, eine verbesserte Bilanzierung von Flüssigkeitsströmen bereitzustellen.

### Zusammenfassung der Erfindung

Diese Aufgabe wird gelöst durch ein Kassettenmodul nach Anspruch 1, sowie durch einen Differenzflussmesser nach Anspruch 5. Vorteilhafte Weiterbildungen sind in den abhängigen Ansprüchen angegeben.

Im Einklang mit der Lehre der vorliegenden Erfindung wird ein Kassettenmodul für einen Differenzflussmesser bereitgestellt. Der Differenzflussmesser enthält in einer vorteilhaften Weiterbildung:
ein Kassettenmodul mit einem ersten und einem zweiten Kanal,
einen Magneten zum Erzeugen eines magnetischen Feldes in dem ersten und in dem zweiten Kanal,
mit einem jeweils einem an dem Kassettenmodul angeordneten Elektrodenpaar zum Ableiten einer elektrischen Spannung an dem ersten und an dem zweiten Kanal, wenn die Flüssigkeit durch den ersten beziehungsweise durch den zweiten Kanal fließt, so dass ein Unterschied der abgeleiteten Spannungen eine Differenz zwischen einem Flüssigkeitsstrom durch den ersten und durch den zweiten Kanal anzeigt.

Das Kassettenmodul ist dergestalt ausgebildet, dass eine geometrische Verformung der Kanäle aufgrund einer Temperaturdifferenz zwischen dem ersten und dem zweiten Kanal weitgehend ausgeschlossen oder mindestens minimiert wird. Dadurch dass eine geometrische Verformung der Kanäle ausgeschlossen oder minimiert wird, bleiben die Lagebeziehungen zwischen den Elektroden und dem Magnetfeld erhalten, wenn eine Temperaturdifferenz zwischen dem ersten Kanal und dem zweiten Kanal herrscht.

Eine konstante Lagebeziehung zwischen Elektroden und magnetischem Feld trägt zu einer erhöhten Messgenauigkeit des Differenzflussmessers bei.

Eine Verformung der Kanäle im Betrieb des Differenzflussmessers kann dadurch im Wesentlichen ausgeschlossen oder vermindert werden, dass das Kassettenmodul einen Grundkörper bildet, an dem die fluidführenden Kanäle angebracht sind, wobei die geometrische Verformung durch eine Versteifungsstruktur des Grundkörpers minimiert wird. Die Versteifungsstruktur ist vorteilhaft dadurch gegeben, dass der Grundkörper massiv ausgebildet ist und somit der Grundkörper selbst eine Versteifungsstruktur bildet. In einer alternativen Ausführungsform des Kassettenmoduls wird die Versteifungsstruktur durch Versteifungsrippen gebildet.

Eine Verformung der Kanäle kann nicht erfindungsgemäß beispielsweise dadurch ausgeschlossen oder minimiert werden, dass zwischen den Kanälen eine Isolationsschicht zur thermischen Isolation der Kanäle vorgesehen ist. Die Isolationsschicht kann aus einem Isolatormaterial, das als thermischer Isolator wirkt, ausgeführt sein, beispielsweise aus Keramik oder aus einem Schaumstoff. In einer alternativen Ausführungsform ist zwischen den Kanälen eine Aussparung vorgesehen, so dass die Kanäle durch die zwischen ihnen liegende Luftschicht isoliert sind.

In einer vorteilhaften Ausführungsform des Kassettenmoduls beträgt die aufgrund der Verformung der Kanäle auftretende Messwertänderung weniger als 0,05 Prozent pro Kelvin Temperaturdifferenz zwischen den Kanälen.

In einer weiteren nicht erfindungsgemäßen Ausführungsform wird die geometrische Verformung dadurch minimiert oder verhindert, dass eine Kontaktzone zwischen dem ersten und dem zweiten Kanal minimiert wird. Als Kontaktzone wird der Bereich der Kanäle angesehen, in dem der erste und der zweite Kanal sich nahe kommen. Dieser Bereich wird im Allgemeinen der Bereich sein, in dem das gemeinsame Magnetfeld den ersten und den zweiten Kanal durchdringt und in dem die Elektrodenpaare angeordnet sind.

Die Kontaktzone zwischen dem ersten und dem zweiten Kanal wird beispielsweise dadurch minimiert, dass der erste und der zweite Kanal im Wesentlichen senkrecht zu einander angeordnet sind.

Erfindungsgemäß sind mindestens zwei erste Kanäle und mindestens zwei zweite Kanäle vorgesehen, die Kanäle sind derart angeordnet, dass sich mechanische Spannungen aufgrund einer Temperaturdifferenz zwischen den ersten und den zweiten Kanälen ausgleichen.

Beispielsweise können zwei erste Kanäle für eine Strömungsrichtung und zwei zweite Kanäle für die entgegengesetzte Strömungsrichtung vorgesehen sein, wobei die insgesamt vier Kanäle parallel zu einander angeordnet sind und sich die beiden ersten Kanäle und die beiden zweiten Kanäle jeweils gegenüberliegen.

Eine solche Anordnung führt auch dann zu keinen oder nur geringen Verformungen, wenn keine besondere Verstärkungsstruktur vorgesehen ist. Eine solche Anordnung ist daher besonders materialsparend.

Das Kassettenmodul kann Teil eines Dialysierflüssig-keitskreislaufs sein und der Differenzflussmesser kann zur Bilanzierung von Dialysierflüssigkeit zwischen einem Zustrom von frischer Dialysierflüssigkeit zu einer Blutbehandlungseinheit und einem Ablauf für verbrauchte Dialysierflüssigkeit vorgesehen sein. Beispielhaft enthält dass Kassettenmodul weitere Elemente eines Dialysierflüssigkeitskreislaufs, wie etwa eine Dialysierflüssigkeitspumpe.

In einer vorteilhaften Ausführungsform ist dass Kassettenmodul vorgesehen zur Verwendung als Einmalartikel.

### Kurzbeschreibung der Zeichnungen

Fig. 1 - 5 zeigen jeweils nicht erfindungsgemäße Ausführungsformen eines Kassettenmoduls für einen Differenzflussmesser.
Fig. 6 zeigt eine erfindungsgemäße Ausführungsform eines Kassettenmoduls für einen Differenzflussmesser.

### Detaillierte Beschreibung

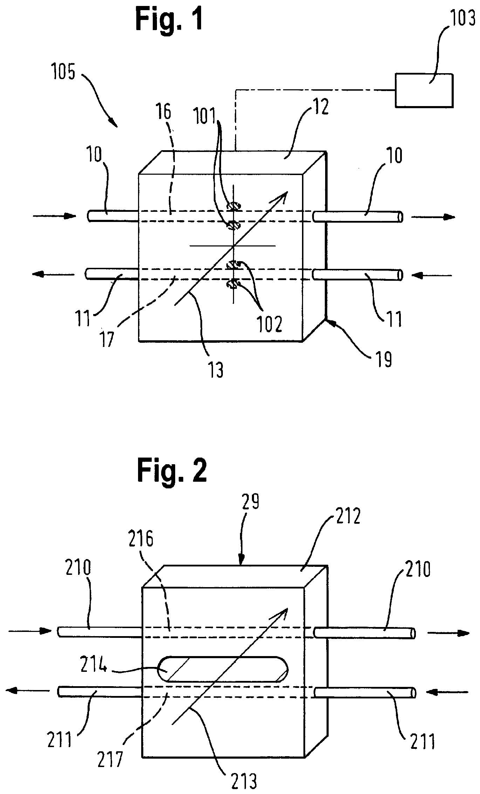

Fig. 1 zeigt ein nicht erfindungsgemäßes Beispiel eines Kassettenmoduls 19 für einen Differenzflussmesser. Das Kassettenmodul 19 weist einen massiven Grundkörper 12 auf, in dem Kanäle 16, 17 ausgebildet sind, die fluidführend sind, wenn der Differenzflussmesser betrieben wird. An die fluidführenden Kanäle 16, 17 schließen sich fluidführende Leitungen 10, 11 an. In einer Ausführungsform ist der Differenzflussmesser Teil einer Bilanziereinheit 105 zum Bilanzieren von Dialysierflüssigkeit in einem Dialysierflüssigkeitskreislauf. In diesem Fall sind die Leitungen 10, 11 sowie die Kanäle 16, 17 Teil eines Dialysierflüssigkeitskreislaufs.

Die Kanäle 16, 17 sind von einem Magnetfeld 13 durchdrungen, das von einem nicht dargestellten Magneten erzeugt wird. Das Magnetfeld steht im Wesentlichen senkrecht zu der Strömungsrichtung in den Kanälen 16, 17. Im Wesentlichen senkrecht zu dem Magnetfeld 13 sowie zu der Strömungsrichtung in den Kanälen 16, 17 sind an den Kanälen 16, 17 Elektroden 101, 102 angeordnet, um eine Spannung quer zur jeweiligen Strömungsrichtung abzugreifen. Die Elektroden sind mit einer Auswerteeinheit 103 verbunden, die aufgrund der abgeleiteten Spannungen, insbesondere aufgrund einer Differenz der abgeleiteten Spannungen eine Differenz der Flüssigkeitsflüsse in den Kanälen und somit eine Flüssigkeitsbilanz zwischen den fluidführenden Leitungen bestimmt.

Durch die massive Ausgestaltung des Grundkörpers 12 ist eine Versteifungsstruktur gegeben, die eine geometrische Verformung des Grundkörpers aufgrund einer Temperaturdifferenz zwischen den Leitungen 10, 11 minimiert. Alternativ kann eine Versteifungsstruktur auch durch Versteifungsrippen gegeben sein.

Fig. 2 zeigt ein weiteres nicht erfindungsgemäßes Beispiel eines Kassettenmoduls 29 für einen Differenzflussmesser. Dass Kassettenmodul 29 weist einen Grundkörper 212 auf, in dem Kanäle 216, 217 ausgebildet sind, die fluidführend sind, wenn der Differenzflussmesser betrieben wird. An die fluidführenden Kanäle 216, 217 schließen sich fluidführende Leitungen 210, 211 an. Die Kanäle 216, 217 sind von einem Magnetfeld 213 durchdrungen. Durch das Magnetfeld 213 induzierte und an nicht näher dargestellten Elektroden abgeleitete Spannungen ermöglichen die Bestimmung einer Flüssigkeitsbilanz, entsprechend der bereits im Zusammenhang mit Figur 1 beschriebenen Anordnung.

Der Grundkörper bildet eine Aussparung 214 zwischen den fluidführenden Kanälen 216 und 217, die eine Isolationsschicht zur thermischen Isolation der Kanäle bildet. Die Aussparung kann mit einem thermischen Isolationsmaterial, wie etwa mit Keramik oder mit einem Schaumstoff ausgefüllt sein. In einer alternativen Ausführungsform ist eine Isolation der Kanäle dadurch gegeben, dass die zwischen den Kanälen liegende Luftschicht isolierend wirkt. Die Isolation zwischen dem Kanal 216 und dem Kanal 217 bewirkt, dass eine geometrische Verformung des Grundkörpers aufgrund einer Temperaturdifferenz zwischen den Leitungen 216 und 217 minimiert wird.

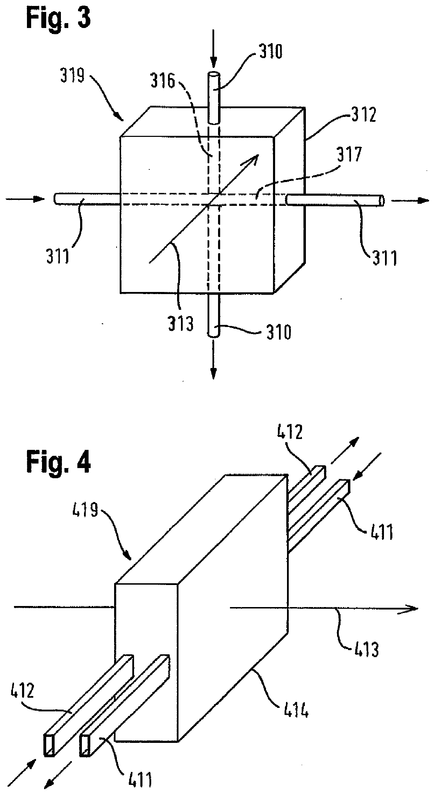

Fig. 3 zeigt ein weiteres nicht erfindungsgemäßes Beispiel eines Kassettenmoduls 319 für einen Differenzflussmesser. Das Kassettenmodul 319 weist einen Grundkörper 312 auf, in dem Kanäle 316, 317 ausgebildet sind, die fluidführend sind, wenn der Differenzflussmesser betrieben wird. An die fluidführenden Kanäle 316, 317 schließen sich fluidführende Leitungen 310, 311 an. Ein von einem nicht darge-stellten Magneten erzeugtes Magnetfeld durchdringt den ersten und den zweiten Kanal in einer Kontaktzone. In diesem Bereich sind auch Elektronen zum Ableiten von an den Elektronen induzierten Spannungen angeordnet. An den Elektroden abgeleitete Spannungen ermöglichen die Bestimmung einer Flüssigkeitsbilanz, entsprechend der bereits im Zusammenhang mit Figur 1 beschriebenen Anordnung.

Die Kontaktzone zwischen dem ersten und dem zweiten Kanal wird dadurch minimiert oder verhindert, dass der erste und der zweite Kanal im Wesentlichen senkrecht zu einander angeordnet sind.

Die minimierte Kontaktzone zwischen dem Kanal 316 und dem Kanal 317 bewirkt, dass eine geometrische Verformung des Grundkörpers aufgrund einer Temperaturdifferenz zwischen den Leitungen 216 und 217 minimiert wird.

Figur 4 zeigt eine alternative nicht erfindungsgemäße Gestaltung eines Kassettenmoduls 414 für einen Differenzflussmesser. Das Kassettenmodul 414 weist einen Grundkörper auf, in dem ein erster und ein zweiter Kanal ausgebildet sind, die fluidführend sind, wenn der Differenzflussmesser betrieben wird. Die Kanäle sind Teil der fluidführenden Leitungen 411, 412 und sind von einem Magnetfeld 413 durchdrungen, das von einem nicht dargestellten Magneten erzeugt wird. Das Magnetfeld steht im Wesentlichen senkrecht zu der Strömungsrichtung in den Kanälen.

Durch das Magnetfeld 413 induzierte und an nicht näher dargestellten Elektroden abgeleitete Spannungen ermöglichen die Bestimmung einer Flüssigkeitsbilanz, entsprechend der bereits im Zusammenhang mit Figur 1 beschriebenen Anordnung.

Eine bestimmte Magnetfeldlinie durchdringt sowohl den ersten als auch den zweiten Kanal. Daher herrscht in dem ersten Kanal im Wesentlichen dasselbe Magnetfeld wie in dem zweiten Kanal. Somit ist eine besonders genaue Bestimmung der Flüssigkeitsbilanz möglich.

Das Kassettenmodul entspricht insofern dem im Zusammenhang mit Figur 1 beschriebenen Kassettenmodul, als dass der Grundkörper massiv ausgestaltet ist. Durch die massive Ausgestaltung des Grundkörpers ist eine Versteifungsstruktur gegeben, die eine geometrische Verformung des Grundkörpers aufgrund einer Temperaturdifferenz zwischen den Leitungen 411, 412 minimiert.

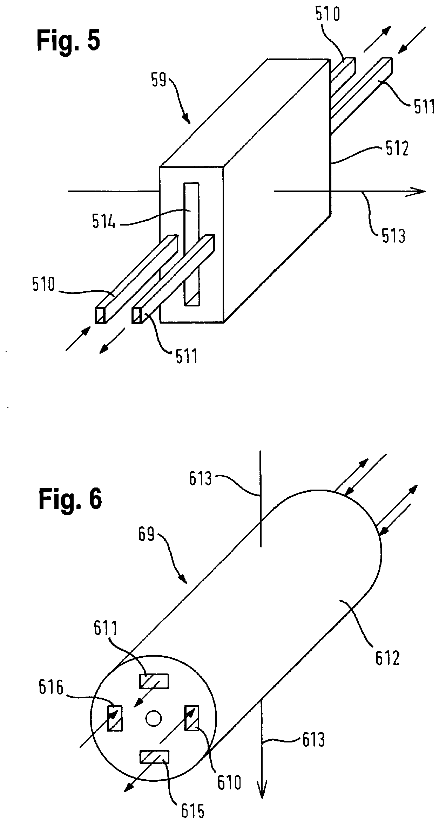

Figur 5 zeigt eine weitere nicht erfindungsgemäße Ausgestaltung eines Kassettenmoduls 9 für einen Differenzflussmesser. Das Kassettenmodul weist einen Grundkörper 512 auf, der einen ersten und einen zweiten Kanal ausbildet, die jeweils Teil der fluidführenden Leitungen 510, 511 sind.

Die Kanäle sind von einem Magnetfeld 513 durchdrungen, das von einem nicht dargestellten Magneten erzeugt wird. Das Magnetfeld steht im Wesentlichen senkrecht zu der Strömungsrichtung in den Kanälen.

Durch das Magnetfeld 513 induzierte und an nicht näher dargestellten Elektroden abgeleitete Spannungen ermöglichen die Bestimmung einer Flüssigkeitsbilanz, entsprechend der bereits im Zusammenhang mit Figur 1 beschriebenen Anordnung.

Eine bestimmte Magnetfeldlinie durchdringt sowohl den ersten als auch den zweiten Kanal. Daher herrscht in dem ersten Kanal im Wesentlichen dasselbe Magnetfeld wie in dem zweiten Kanal. Somit ist eine besonders genaue Bestimmung der Flüssigkeitsbilanz möglich.

Das Kassettenmodul weist eine Aussparung 514 in dem Grundkörper 512 auf, zur thermischen Isolation der Kanäle, Die Aussparung kann mit einem thermischen Isolationsmaterial, wie etwa mit Keramik oder mit einem Schaumstoff ausgefüllt sein. Alternativ ist eine Isolation der Kanäle dadurch gegeben, dass die zwischen den Kanälen liegende Luftschicht isolierend wirkt.

Die Isolation zwischen dem ersten Kanal und dem zweiten Kanal bewirkt, dass eine geometrische Verformung des Grundkörpers aufgrund einer Temperaturdifferenz zwischen den Leitungen 510 und 511 minimiert wird.

Figur 6 zeigt eine erfindungsgemäße Ausgestaltung eines Kassettenmoduls 612 für einen Differenzflussmesser. Das Kassettenmodul 69 weist einen Grundkörper 612 auf, in dem erste Kanäle 611 und 615 sowie zweite Kanäle 610, 616 ausgebildet sind, die fluidführend sind, wenn der Differenzflussmesser betrieben wird. Die ersten Kanäle sind Teil einer ersten im Betrieb des Differenzflussmessers fluidführenden Leitung, die sich in die beiden ersten aufspaltet, und die zweiten Kanäle sind Teil einer zweiten im Betrieb des Differenzflussmessers fluidführenden Leitung, die sich in die beiden zweiten Kanäle aufspaltet.

D.h. die Strömungsrichtungen in den beiden ersten Kanälen entsprechen einander und die Strömungsrichtungen in den beiden zweiten Kanälen entsprechen einander. Durch jeweils gegenüberliegende Kanäle strömt das Fluid in derselben Richtung.

Die Anordnung der Kanäle bewirkt, dass sich mechanische Spannungen aufgrund einer vorhandenen Temperaturdifferenz zwischen der ersten Leitung und der zweiten Leitung entsprechend einer Temperaturdifferenz zwischen den beiden ersten Kanälen und den beiden zweiten Kanälen ausgleichen.

Die Kanäle 611, 610, 616, 615 sind von einem Magnetfeld 613 durchdrungen, das von einem nicht dargestellten Magneten erzeugt wird.

Das Magnetfeld steht im Wesentlichen senkrecht zu der Strömungsrichtung in den Kanälen 611, 610, 616, 615.

Im Wesentlichen senkrecht zu dem Magnetfeld 613 sowie zu der Strömungsrichtung in den Kanälen 611, 610, 616, 615 sind an den Kanälen 611, 610, 616, 615 jeweils Elektroden (nicht dargestellt) angeordnet, um eine Spannung quer zur jeweiligen Strömungsrichtung abzugreifen.

Die Elektroden sind mit einer Auswerteeinheit (nicht dargestellt) verbunden, die aufgrund der abgeleiteten Spannungen eine Differenz zwischen dem Flüssigkeitsfluss in der ersten Leitung und dem Flüssigkeitsfluss in der zweiten Leitung und somit eine Flüssigkeitsbilanz zwischen der ersten und der zweiten Leitung anzeigen.

Dabei werden an den Elektroden der ersten Kanäle abgeleitete Spannungen jeweils addiert um eine Gesamtspannung an den beiden ersten Kanälen zu erhalten und an den Elektroden der zweiten Kanäle abgeleitete Spannungen werden addiert, um eine Gesamtspannung an den beiden zweiten Kanälen zu erhalten. Die beiden Gesamtspannungen werden dann voneinander subtrahiert.

In einer alternativen Ausführungsform sind jeweils nur einem der ersten Kanäle und an einem der zweiten Kanäle Elektrodenpaare vorgesehen.

Eine solche Anordnung führt auch dann zu keinen oder nur geringen Verformungen, wenn keine besondere Verstärkungsstriktur vorgesehen ist. Eine solche Anordnung ist daher besonders materialsparend.

In einer vorteilhaften Ausführungsform des Kassettenmoduls beträgt die auf die Verformung der Kanäle zurückgehende Messwertänderung weniger als 0,05 Prozent pro Kelvin.

Dadurch, dass eine geometrische Verformung der Kanäle verhindert oder minimiert wird, bleiben die Lagebeziehungen zwischen den Elektroden und dem Magnetfeld erhalten, wenn eine Temperaturdifferenz zwischen der ersten und der zweiten Leitung herrscht. Eine konstante Lagebeziehung zwischen Elektroden und magnetischem Feld trägt zu einer verbesserten Messgenauigkeit des Differenzflussmessers bei.

## Patentansprüche

1. Kassettenmodul (69) ausgeführt für einen Differenzflussmesser, wobei das Kassettenmodul (69) einen ersten (611, 615) und einen zweiten (610, 616) im Betrieb des Differenzflussmessers fluidführenden Kanal ausbildet, **dadurch gekennzeichnet, dass** das Kassettenmodul derart ausgeführt ist, dass eine geometrische Verformung der Kanäle durch eine Temperaturdifferenz zwischen den Kanälen (611, 615, 610, 616) minimiert oder verhindert wird indem mindestens zwei erste Kanäle (611, 615) und mindestens zwei zweite Kanäle (610, 616) vorgesehen sind und indem die Kanäle derart angeordnet sind, dass sich mechanische Spannungen aufgrund einer Temperaturdifferenz zwischen den ersten (611, 615) und den zweiten (610, 616) Kanälen ausgleichen.

2. Kassettenmodul (69) nach Anspruch 1, wobei die mindestens zwei ersten Kanäle (611, 615) und die mindestens zwei zweiten Kanäle (610, 616) jeweils spiegelsymmetrisch zu einander angeordnet sind.

3. Kassettenmodul (59, 69) nach einem der vorangehenden Ansprüche, wobei eine auf eine Verformung der Kanäle zurückgehende Messwertänderung weniger als 0,05 Prozent pro Kelvin beträgt.

4. Kassettenmodul (59, 69) nach einem der vorangehenden Ansprüche zur Verwendung als Einmalartikel.

5. Differenzflussmesser (105) zur Bilanzierung zwischen Flüssigkeitsströmen enthaltend:
ein Kassettenmodul (59, 69) nach einem der Ansprüche 1 - 4,
einen Magneten zum Erzeugen eines magnetischen Feldes in dem ersten (611, 615) und in dem zweiten Kanal (610, 616) des Kassettenmoduls, jeweils ein Elektrodenpaar (101, 102) zum Ableiten einer elektrischen Spannung an dem ersten (611, 615) und an dem zweiten (610, 616) Kanal,
wenn Flüssigkeit durch den ersten beziehungsweise durch den zweiten Kanal fließt, so dass ein Unterschied der abgeleiteten Spannungen eine Differenz zwischen einem Flüssigkeitsstrom durch den ersten (216, 611, 615) und durch den zweiten (610, 616) Kanal anzeigt.

6. Differenzflussmesser (105) nach Anspruch 5 mit einer Auswerteeinheit (103) zur Bestimmung der Differenz zwischen der an der ersten (611, 615) und der an der zweiten (610, 616) fluidführenden Kanal abgeleiteten Spannung und zur Bestimmung der Differenz der Flüssigkeitsflüsse basierend auf der bestimmten Spannungsdifferenz.

## Claims

1. A cassette module (69) embodied for a differential flowmeter, wherein the cassette module (69) forms a first (611, 615) and a second (610, 616) channel, which conducts fluid during operation of the differential flowmeter, **characterized in that** the cassette module is embodied in such a way that a geometric deformation of the channels due to a temperature difference between the channels (611, 615, 610, 616) is minimized or prevented **in that** at least two first channels (611, 615) and at least two second channels (610, 616) are provided, and **in that** the channels are arranged in such a way that mechanical stresses due to a temperature difference between the first (611, 615) and the second (610, 616) channels compensate one another.

2. The cassette module (69) according to claim 1, wherein the at least two first channels (611, 615) and the at least two second channels (610, 616) are each arranged mirror-symmetrically to one another.

3. The cassette module (59, 69) according to one of the preceding claims, wherein a change in the measured value, which is caused by a deformation of the channels, is less than 0.05 percent per Kelvin.

4. The cassette module (59, 69) according to one of the preceding claims for use as disposable article.

5. A differential flowmeter (105) for balancing between liquid flows including:
a cassette module (59, 69) according to one of claims 1-4,
a magnet for generating a magnetic field in the first (611, 615) and in the second channel (610, 616) of the cassette module,
an electrode pair (101, 102) each for dissipating electrical voltage on the first (611, 615) and on the second (610, 616) channel,
when liquid flows through the first or through the second channel, respectively, so that a difference of the dissipated voltages indicates a difference between a liquid flow through the first (216, 611, 615) and through the second (610, 616) channel.

6. The differential flowmeter (105) according to claim 5, comprising an evaluating unit (103) for determining the difference between the voltage dissipated on the first (611, 615) fluid-conducting channel and the voltage dissipated on the second (610, 616) fluid-conducting channel, and for determining the difference of the liquid flows based on the determined voltage difference.

## Revendications

1. Module de cassette (69) réalisé pour un débitmètre différentiel, le module de cassette (69) constituant un premier (611, 615) et un second (610, 616) canal conducteur de fluide en cours de fonctionnement du débitmètre différentiel, **caractérisé en ce que** le module de cassette est réalisé de manière à ce qu'une déformation géométrique des canaux par une différence de température entre les canaux (611, 615, 610, 616) soit minimisée ou empêchée du fait qu'au moins deux premiers canaux (611, 615) et au moins deux seconds canaux (610, 616) sont prévus et que les canaux sont disposés de manière à ce que les tensions mécaniques dues à une différence de température entre les premiers (611, 615) et les seconds (610, 616) canaux s'équilibrent.

2. Module de cassette (69) selon la revendication 1, dans lequel les au moins deux premier canaux (611, 615) et les au moins deux seconds canaux (610, 616) sont disposés respectivement symétriques en miroir l'un par rapport à l'autre.

3. Module de cassette (59, 69) selon une des revendications précédentes, dans lequel une modification de valeur de mesure due à une déformation des canaux est de moins de 0,05 % par kelvin.

4. Module de cassette (59, 69) selon une des revendications précédentes, destiné à une utilisation comme article à usage unique.

5. Débitmètre différentiel (105) destiné à l'équilibrage entre des flux de liquide, contenant :
un module de cassette (59, 69) selon une des revendications 1 à 4,
un aimant pour la génération d'un champ magnétique dans le premier (611, 615) et dans le second (610, 616) canal du module de cassette,
respectivement une paire d'électrodes (101, 102) pour l'évacuation d'une tension électrique au niveau du premier (611, 615) et du second (610, 616) canal
lorsque du liquide s'écoule à travers le premier ou à travers le second canal, de sorte qu'une différence de tensions évacuées indique une différence entre un flux de liquide traversant le premier (216, 611, 615) et le second (610, 616) canal.

6. Débitmètre différentiel (105) selon la revendication 5, comportant une unité d'exploitation (103) pour déterminer la différence entre la tension évacuée au niveau du premier (611, 615) et du second (610, 616) canal conducteur de fluide et pour déterminer la différence entre les flux de liquide en se basant sur la différence de tension définie.
